# EUROPEAN PATENT SPECIFICATION

(11) **EP 1 503 656 B1**
(45) Date of publication and mention of the grant of the patent: **02.03.2011**
(21) Application number: 03722646.1
(22) Date of filing: 05.05.2003
(51) Int. Cl.: A61B 3/10, A61B 5/04

(54) **ELECTRODE DETECTOR FOR MONITORING BIOPOTENTIAL ON TISSUE**
ELEKTRODENDETEKTOR ZUR ÜBERWACHUNG DES BIOPOTENTIALS AN GEWEBE
DETECTEUR A ELECTRODE POUR LE CONTROLE DU BIOPOTENTIEL D'UN TISSU

(30) Priority: 06.05.2002 FI 20020850
(43) Date of publication of application: 09.02.2005
(73) Proprietor: Valjakka, Antti, 20320 Turku (FI)
(72) Inventor: VALJAKKA, Antti, 71570 Kuopio (FI); URTTI, Arto, 70420 Kuopio (FI); AHONEN, Janne, 70260 Kuopio (FI)
(74) Representative: Pitkänen, Hannu Alpo Antero
(86) International application number: PCT/FI2003/000352
(87) International publication number: WO 2003/092484

(56) References cited:
- US-A- 4 735 207
- US-A- 5 154 174
- US-B1- 6 208 882

## Description

The present invention relates to an electrode detector in accordance with the introduction of claim 1.

In visual measurement methods based on a recognized technique which methods have been designed for measuring the physiological potential difference in the retina of the eye (ERG, electroretinogram) for visual stimulus on test animals the electrode types referred to and to be adjusted in the eye and which electrodes registrate the ERG response have been constructed such that the use of those requires anaesthesia or immobility of the test animal such that the contact part of the electrodes measuring the biopotential is well kept on place and that they could in such way measure ERG responses of the same size and without interference. For example, the thread electrode, the cotton wick-Ag/AgCl-electrode and a contact lens with golden coat -electrode described in publication Documenta Ophthalmologica 98: 2000, Bayer A.U., Mittag T., Cook P., Brodie S.E., Podos S.M. and Maag K-P. "Comparisons of the amplitude size and the reproducibility of three different electrodes to record the corneal flash electroretinogram in rodents", p. 233-246, and penlike pointer tip including a light source for stimulation of the retina and the electrode blades for measuring the ERG response described in SU-patent publication 1648336 are these kind of recognized electrode types adjustable on the cornea of the eye.

On the other hand, also other recognized electrodes which have been designed for measuring the ERG response on a human, but which, in some cases, may be applied for monitoring test animals are designed by shape such that their use requires the subject to be immovable during the measurements. Electrodes described in these methods are:
(1) A looplike, so called, HK-loop electrode for clinical ERG-measurements (Documenta Ophthalnlologica 81:1992, Hawlina M. and Konec B. "New noncorneal HK-loop electrode for clinical electroretinography", p. 253-259; US-patent 5154174)
(2) A cluster of electrodes placed on the sclerotic membrane of eye, in which electrode cluster there are several separate contact blades of electrodes sank in a ringlike and concave insulation frame following the surface of the eye for use in clinical and experimental (that is for test animals) ERG measurements (US-patent 4874237).
(3) A contact lens kind of device placed on the corneal membrane of the eye for clinical monitoring of functioning of the cardiovascular system and ERG (US-patent 5297554).
(4) A nylon fibre electrode coated with silver and adjustable on the corneal membrane of the eye for clinical ERG registrations (US-patent 4417581).
(5) A thin, membrane-type, mold to desired form electrode including water to be attached on the corneal membrane for applying in clinical and experimental ERG measurements (US-patent 4735207). US-patent 4 735 207 discloses an electrode detector according to the preamble of claim 1.
(6) An electroretinografic transparent coat with taps holding the eyelids open to be adjusted on the corneal membrane for applying in clinical and experimental ERG measurements (FR patent publication 2713913).
(7) A metal strip to be attached on the skin, on the lower eyelid for example, which metal strip has been connected to a flexible plastic strip for measuring ERG and the outside cranium-reflected responses on a human (US patent 4255023).
(8) A transparent cup electrode to be attached on the corneal membrane, on the edges of which cup electrode there is a metal ring for defining ERG on a human (US patent 4131113).
(9) A metal wire to be attached on the lower eyelid on the skin for clinical ERG registrations (US patent 5506633).

Most of these referred techniques enable the registration of ERG and other visual responses reflected outside the cranium on a conscious human, because a test person is able to stay immovable deliberately. Applying above mentioned techniques in monitoring test animals is difficult in that with registration of ERG on test animals under anaesthesia or made immovable the kind of drugs have to be used which change the functioning of cells of the central nerve system and thus the normal physiology of seeing.

The common limiting factor of recognized electrode detectors designed for visual monitoring is their shape which may cause irritation changes in the eye or in the surrounding tissue or excessive stress on the eye if they were implanted permanently on the eye, for example, under the eyelid. Secondly, a limiting factor which excludes their permanent implantation in the eye is the fact that the electrode conductors have been described to be directed outwards from the eye where they have been presented to be connected to the actual measuring system. The third problem connected with recognized electrode detectors is the fact how the contact part measuring the biopotential and being connected to the tissue is kept on place while changing stresses are directed to it through the eye and the tissue around it. On the other hand, a problem connected with earlier mentioned is the fact that no stresses ought to be directed to the contact part of the electrode due to possible movements of the measuring wire.

The object of the invention is to eliminate these problems and to provide an electrode detector, which is (a) such by construction that the contact electrode part measuring the biopotential is fixed to hold on desired place, (b) the contact electrode part yields under mechanical stress directed to it and returns to its place by a little movement, (c) the electrode detector does not cause mechanical or chemical tissue injury and (d) mechanical stresses of the measuring wire which is possible to be connected to the electrode detector are not transmitted to the contact electrode part.

The object of the invention is accomplished by an electrode detector, the characteristics of which are presented in claim 1.

The electrode detector in accordance with the invention is defined in claim 1. It comprises a ball-shaped contact electrode part monitoring biopotential or some other physical magnitude, the covering part of which contact electrode part is made of non-toxic, material to tissue and some electroconductive part of which is connected through an electronic circuitry in the contact electrode part to the electroconductive inner part of a flexible conducting wire while its covering part is made of material that is non-toxic to tissue and isolates electricity, and the attaching part, which is connected through the electronic circuitry in the attaching part, to the conducting wire of the electrode detector, and which may be anchored with external attaching elements/attaching material or attaching elements/material in it, near the object to be monitored.

The ball-shaped form of the contact electrode part in accordance with the invention does not cause mechanical wearing injuries or irritation changes while placed in living biological tissue or while placed against such. The contact electrode part measuring biosignal keeps on place when the conducting wire penetrates a space which the contact electrode part and the attaching part do not penetrate and when the attaching part may be anchored on place with attaching elements. The ball shaped contact electrode part does not direct too much stress on the object to be measured, due to movements of the tissue, for example, because while connected to a flexible wire it yields. While the attaching part may be anchored with attaching elements in it or with external attaching parts the movements of the measuring wire connected to it are not transmitted to the contact electrode part and the signal processing with an electronic circuitry in the electrode detector itself minimizes the relative proportion of interference signals with respect to biosignal.

The earlier mentioned purposes, for measuring the ERG biopotential response, for example, are achieved by employing the ball-shaped contact electrode part in accordance with the invention which contact electrode part may be surgically implanted to a desired place in tissue, such as under the eyelid, against the sclerotic membrane or whatever suitable place in the eye or non-invasively externally on tissue to a suitable place. An electrode detector with earlier mentioned properties enables, among other things, evaluation of functioning of the eye and indirectly the optic nerve of eye of a freely moving and conscious test animal in measurements during various points of time, for example, during several months, without surgical operations in connection with these measurements. Naturally, in certain conditions, with these kinds of electrode detectors it is possible to measure the ERG in a human eye. An electrode detector meeting the earlier mentioned requirements may be applied as well in measurements of other physical magnitudes, such as temperature, conductibility, pressure in a monitored object while the measurement problem is as described in the introduction.

A contact electrode, the size of which depends on the subject to be measured, may be made of whatever material with good conductivity of electricity, preferably of pure precious metal. In cases, where electroconductive material is toxic to tissue it may be coated with electroconductive (pure silver, for example) or non-electroconductive material (Teflon-plastic, for example) which has no toxic influence on tissue. The biopotential change, caused by tissue and taken place in contact electrode part is transmitted to the electric wire attached to it, which is possible to place inside tissue while coated with insulation that is non-toxic to tissue, such as Teflon or similar.

A conducting wire has been attached or is connectable immovably to the attaching part, which may be anchored on place, near the object to be measured, by means of attaching elements on it and/or by means of external attaching elements/materials. The attaching part may be cast into plastic, for example, or similar material isolating electricity and being non-toxic to tissue. The attaching part may be anchored either inside tissue by means of bolts and hardening cold acrylic or externally by using a glue sticker or a suction cup. In cases when an electrode detector is planned to be placed against tissue, outside the body, the attaching part may be of flexible, gelatinous material (rubber, for example) which, in desired amount, surrounds the whole electrode detector. The attaching part may include electric connectors to the conducting wire of the contact electrode, to the wire to be connected to the registration system and to electronic component to be connected to it which electronic component processes the biosignal to be measured.

The electronic component may be, in that way, externally connectable with the attaching part. The electronic circuitry processing the signal may also be located inside the contact electrode part and/or attaching part. In an example, where a described electrode detector has been inserted in the eye of a test animal the contact electrode part of the electrode detector measuring the biopotential keeps on place in desired point of the eye such as between the eyelid and the conjunctiva/corneal membrane of the eye. This is realized by (a) the ability of the attaching part to be permanently anchored on place, (b) a conducting wire of the electorde detector with suitable bending strength and ability to return in shape after bending, (c) the location of the wire inside the tissue such that the tissue itself limits the movement sideways, (d) the location of the ball-shape contact electrode part outside the tissue round a thin opening through the tissue where the conducting wire penetrates the tissue, and (e) forces of the eyelid and the conjunctiva or corneal membrane directed to the ball and limiting its movement. Because of these factors the contact electrode part of the electrode detector does not strain the surface of the eye with too strong stress although variable forces would be directed to the contact electrode part due to movements of the eye or tissue muscles because it yields while connected to a flexible conducting wire.

While the attaching part is possible to anchor permanently the movements of the measuring wire connected to it does not transmit stress on the ball-shaped measuring electrode. This and the fact that the biosignal is possible to be processed (with a differential amplifier, for example) in the attaching part or in the contact electrode part themselves, enable the decreasing of interference signals with respect to the biosignal to be measured.

Next, the invention will be explained in more detail with reference to the accompanying drawings, in which,
Figure 1 illustrates an electrode detector in accordance with the invention and measuring ERG,
Figure 2 illustrates another electrode detector in accordance with the invention, and
Figure 3 illustrates an application of the invention for measuring ERG in according to an example.

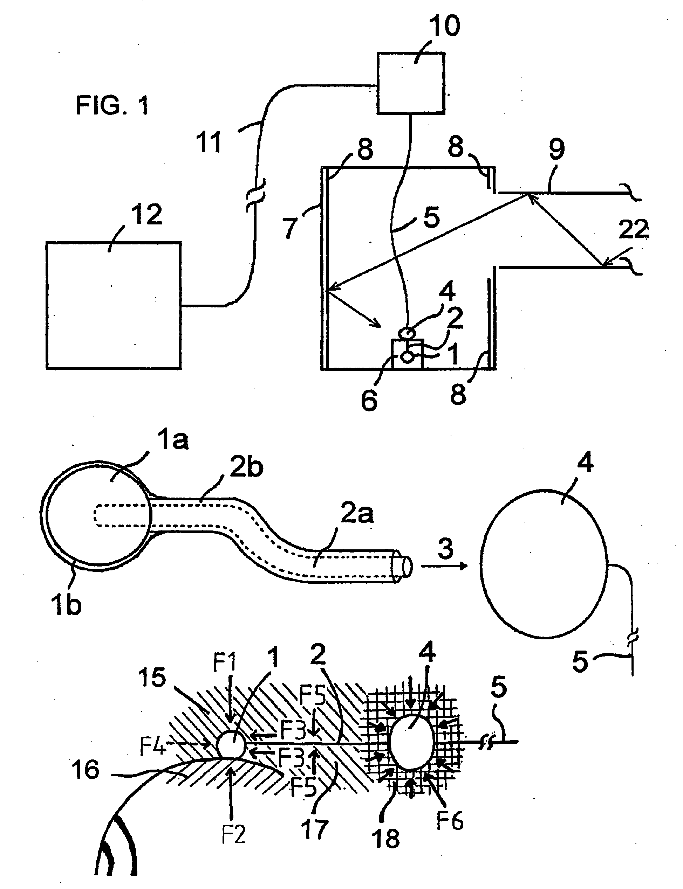

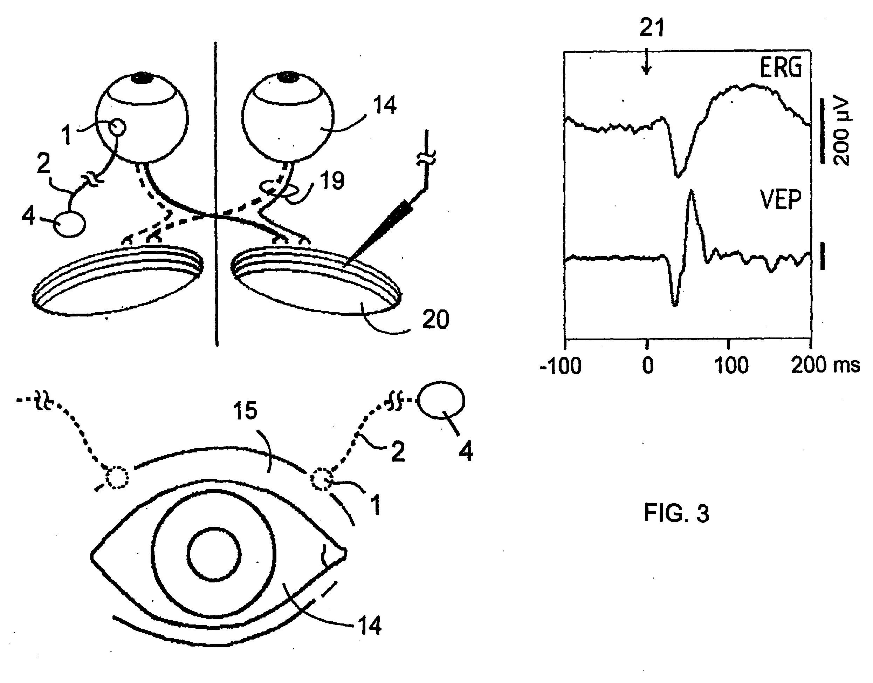

In figures 1 and 3 following numbers refer to various parts of the body: the eye 14, the eyelid 15, the sclerotic membrane of the eye 16, tissue 17, bone 18, the optic nerve of eye 19, superior colliculus 20. Furthermore, in figures following numbers are used: a flashlight 21 and a light beam 22.

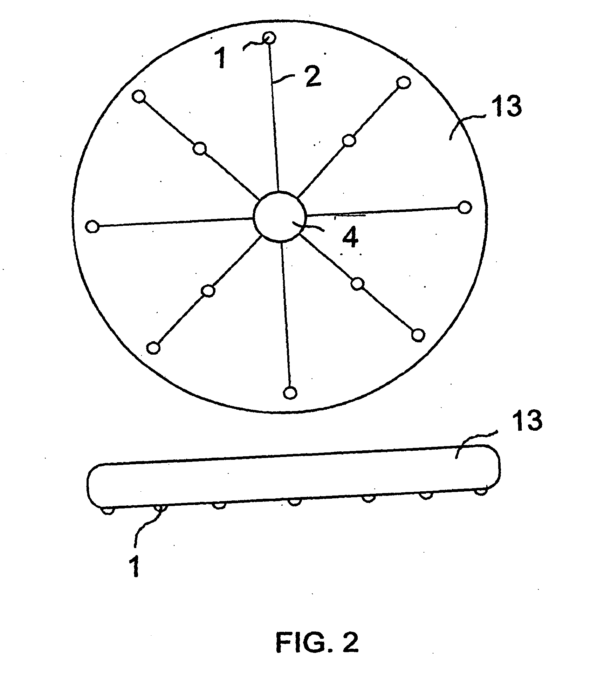

The electrode detector in figures 1 and 2 comprises a conducting wire 2, to one end of which a ball-shaped contact electrode part 1 has been connected. The contact electrode part 1 may be all over of same material such as pure silver, gold, carbon fibre or similar, which is advantageously electroconductive and non-toxic to tissue. On the other hand, the inner part 1a of the contact electrode part 1 may be made of whatever material and of one or several materials and of whatever shape, while being coated to a ball-shape with material which is non-toxic to tissue. For example, while the covering part 1b is of glass, Teflon-plastic or similar, the inner part 1a may be of copper, tin, silver and so on solid and preferable high electroconductive material. In case the covering part 1b of the contact electrode part 1 is an electrical insulation it is preferably commonly continuos with the covering part 2b of the conducting wire 2 (Figure 1B). The area of the contact electrode part 1 may consist of areas bordered by each other and being made of various non-toxic materials, polyethylene polymers and silver, for example. The outer diameter of the contact electrode part 1 may measure 1,2 mm, for example, in ERG measurement on a rat. In the application in figure 2 there is a flexible gelatinous ring from where the ball-like, partly into gelatinous material 13 sunk, contact electrode parts 1 protrude. This kind of electrode detector may be placed against tissue in order to define its physical properties.

In an advantageous application of the invention some material of the inner part 1a of the contact electrode part 1 is in the liquid state. In the second advantageous application of the invention some material of the inner part la of the contact electrode part 1 is in the gaseous state. In the third advantageous application of the invention some part of the inner part la of the contact electrode part 1 is a vacuum, thus making the construction lighter.

The inner part 2a, conducting electricity, of the conducting wire 2 is preferably made of the same material as the material to which it is supposed to be connected in the contact electrode part 1. The inner part 2a of the conducting wire. 2 may be connected to the electroconductive material of the contact electrode part 1 through the electronic circuitry processing the signal, which electronic circuitry is placed in the contact electrode part 1. The inner part 2a of the conducting wire 2 is preferably a multistrand thread braiding in order to have a good mechanical strength. Some or all of the individual braidings may be coated with electrically insulating material. The electroconductive material of the inner part 2a of the conducting wire 2 as well as the electrically insulating material of the covering part 2b are preferably of material returning to its shape after bending. The covering part 2b of the conducting wire 2 must be of non-toxic to tissue material, such as Teflon-plastic or similar. In the conducting wire 2 there may be one or several attaching elements (not illustrated) which are possible to be attached to the attaching part. The proportion of the outer diameter of the contact electrode part 1 to the outer diameter of the conducting wire 2 may be 4:1, for example, or such that the former is clearly bigger than the latter. Round the conducting wire 2 against the covering part 2b of it a free-form and free-size attaching part 4 may be connected, which attaching part 4 may be anchored by means of one or several attaching elements in it (Figure 2) or by means of external attaching elements/material near to the subject to be measured. The attachment may be realized on the cranium, for example by means of bolts and hardening cold acrylic, on the skin, e.g., by means of a glue sticker or a suction cup, or it may be inserted inside tissue in which case the surrounding tissue stabilizes it on place. The material of the attaching part may be wholly or partly flexible and gelatinous substance such as rubber or similar to it material. It may also be of hard material such as glass. The material of the attaching part may reach, in desired extent, round the conducting wire 2 and the contact electrode part 1. For example, half of the ball surface of the contact electrode part 1 may be visible or free from attaching part material.

The outer surface of the attaching part 4 is made of non-toxic to tissue material. There may be an electronic circuitry processing the signal in it. In the attaching part 4 there may be a coupler 3 to be connected to the conducting wire 2, which coupler anchores the conducting wire 2 on place a) by grabbing the covering part 2b of the conducting wire 2 or b) by attaching it to the electroconductive inner part 2a of the conducting wire 2. In the attaching part 4 there may be (not illustrated) an electric coupler for a measuring wire 5 and/or for external (not illustrated) electronic circuitry. The contact electrode part 1 connected to the conducting wire 2 is adjustable to the eye of the test animal 6, for example, by passing the conducting wire 2, in this case, under the upper eyelid inside tissue and conducting it there under skin to a suitable distance where by moving the part of the conducting wire 2 coming outside tissue the contact electrode part 1 may be positioned to the desired point in the eye against the corneal membrane or the conjunctiva (Figure 3).

In the example in accordance with figure 1 the electrode detector is anchored on place by connecting the attaching part 4 outside the skin/tissue to a point, where the conducting wire 2 protrudes from the skin/tissue. The anchoring of the attaching part on the surface of the cranium has been realized by utilizing bolts and hardening acrylic. By employing this technique the construction contact electrode part 1 - the conducting wire 2 - the attaching part 4, makes a unit in which forces F1-3 and F5-6 keep the ball-shaped contact electrode part 1 which is monitoring the biopotential permanently placed in desired point in the eye against the conjunctiva or the corneal membrane (Figure 1). The attaching part 4 limits the movement of the conducting wire 2 in all direction by forces F6 but most of all the longitudinal outward draft F3 of the conducting wire 2 from the attaching part, which is caused by the forces F3 directed to that area of the surface of the contact electrode 1, from the centre of which the conducting wire 2 starts penetrating the tissue through a thin opening which tissue the contact electrode part 1 may not penetrate (Figure 1). Movements of the contact electrode part 1 are limited by forces F5 directed by the tissue to the conducting wire 2 and which are directed perpendicularly to the direction of the conducting wire 2, on one hand, and by forces F 1 and F2 directed by the inner surface of the eyelid and the conjunctiva/corneal membrane of the eye to the contact electrode part 1, on the other hand. As only the attaching part 4 anchors the other end of the conducting wire 2 immovable on place and as the conducting wire 2 is suitably flexible and capable to return after bending back to its shape the contact electrode part 1 does not direct too strong stress on the surface of the eye (Figure 1) due to the movements of the eye and the eyelid, for example, because it yields. An electrode detector in accordance with above described may be applied in measuring the ERG response of the eye on a test animal 6 (a rat, for example) while it is free to move and conscious, for example, in situation where in chamber 7 visual stimulus refers to diffused light reflected with mirrors 8 and 9 and where a measuring wire 5 connected to the attaching part 4 of the electrode detector transmits the measuring information to the data processing unit 12 through an usual signal processing unit 10 and contact wire 11 (Figure 1). By utilizing this technique it is, therefore, possible to evaluate not only the functioning of the eye but also the functioning of the optic nerve on a freely moving test animal while a visual response has been registered from some other point of the visual path, for example from "superior colliculus" structure. (Figures 1 and 3).

In accordance with the idea of the invention the ball-shaped electrode part 1 monitoring biopotential in construction contact electrode 1 - the conducting wire 2 - the attaching part 4 is such, that it does not cause mechanical irritation changes while placed inside tissue. Secondly, the ball-like contact electrode 1 does not direct too strong stress to the subject to be measured which stress could be caused by movements in the surrounding tissue because it yields under the forces directed to it while being connected to the flexible conducting wire 2 which returns to its shape after bending and the other end of which may be anchored permanently on place in suitable distance from the subject to be measured. Thirdly, the ability to be anchored of the attaching part 4 of the electrode detector by utilizing the attaching elements in it or external attaching elements/materials as well as forces (F1-3, F5-6) directed by the surrounding tissue to various sides of the electrode detector cause the ball-shaped contact electrode part 1 to stay on place. Fourthly, the ability to be anchored of the attaching part excludes the transmission of forces directed from the measuring wire 5 to the contact electrode part 1. Fifthly, the parts (not illustrated) of the electrode detector processing the signal, such as the differential amplifier circuitry, decrease the proportion of interference caused by the movements of the measuring wire 5 and reflected from the surroundings with respect to the biosignal to be measured in wire 5.

Within the frames of the invention solutions and applications differing from the earlier described may be considered. And such the electrode detector may alternatively comprise designs in which the number of ball-shaped contact electrode parts 1, the attaching parts 4 and conducting wires 2 or the way they are connected to each other is not limited. For example, two contact electrode 1 - conducting wire 2 units may be connected to the same attaching part 4, or two contact electrode parts 1 connected one after another to the same conducting wire 2 which has been connected to one attaching part 4. The described electrode detector enables the measurement of visual response (ERG) from the eye on a human, while a ball-shaped contact electrode part 1 has been inserted under the lower eyelid against the corneal membrane or on the eyelid and the attaching part 4 has been fixed by means of a glue stick or a suction cup on the lower eyelid or on skin near it.

In accordance with the principle which has been described the electrode detector is appliable for measuring a potential change created by whatever biological subject, or of other physical magnitude where the problems connected with the registration are as described.

The invention is not limited to the presented advantageous application but it can vary within the scope of the claims.

## Claims

1. An electrode detector, which comprises
- a. contact electrode part (1) for monitoring biopotentials on an eye of a person or on an eye of an animal, the contact electrode part (1) comprising a covering part (1b) which is non-toxic to tissue material and an electroconductive part,
- a flexible conducting wire (2) attached to the contact electrode part (1), the fexible conducting wire (2) having an electroconductive inner part (2a) and a covering part (2b) which is non-toxic to tissue and insulates electricity,
- an attaching part (4),
- electronics for processing the signals,
**characterized in that**
- the contact electrode part (1) is ball-shaped,
- the electroconductive part of the contact electrode part (1) is connected through an electronic circuitry in the contact electrode part (1) to the electroconductive inner part (2a) of the flexible conducting wire (2),
- the attaching part (4) is capable of keeping the electrode part attached on the person or the animal, and
- the attaching part (4) has an electronic circuitry for connecting to the conducting wire 1 (2).

2. An electrode detector in accordance with claim 1, **characterized in that** the inner part (1a) of the contact electrode part (1) comprises more than one separate material.

3. An electrode detector in accordance with claim 1, **characterized in that** in the attaching part (4) there is one or more electric couplings.

4. An electrode detector in accordance with claim 1, **characterized in that** in the attaching part (4) there is one or more couplings gribbing the covering part (2b) of the conducting wire (2).

5. An electrode detector in accordance with claim 1, **characterized in that** in the covering part (2b) of the conducting wire (2) attached to the contact electrode part (1) there is one or more attaching elements.

6. An electrode detector in accordance with claim 1, **characterized in that** in the attaching part (4) there is one or more attaching elements.

7. An electrode detector in accordance with claims 1 and 6, **characterized in that** one or more attaching elements of the attaching part (4) are of flexible gelatinous material, such as rubber.

8. An electrode detector in accordance with claims 6 and 7 , **characterized in that** said one or more attaching elements of the attaching part (4) are of a flexible gelatinous material which covers the whole electrode detector or a part of it.

9. An electrode detector in accordance with claim 1, **characterized in that** the electronic circuitry inside the attaching part (4) is a differential amplifier.

10. An electrode detector in accordance with claim 1, **characterized in that** the electronic circuitry inside the contact electrode part (1) is a differential amplifier.

11. An electrode detector in accordance with claim 1, **characterized in that** the attaching part (4) of the electrode detector is an electricity insulating, non-toxic material.

12. An electrode detector in accordance with claim 1, **characterized in that** it comprises additional contact electrode parts(1), conducting wires(2) and attaching parts (4).

## Patentansprüche

1. Elektrodendetektor, umfassend:
- ein Kontaktelektrodenteil (1) zur Überwachung von Biopotenzialen auf einem Auge einer Person oder auf einem Auge eines Tieres, wobei das Kontaktelektrodenteil (1) ein Abdeckteil (1b), das für Gewebematerial nicht toxisch ist, und ein elektrisch leitendes Teil umfasst,
- einen flexiblen leitenden Draht (2), der an dem Kontaktelektrodenteil (1) befestigt ist, wobei der flexible leitende Draht (2) ein elektrisch leitendes Innenteil (2a) und ein Abdeckteil (2b) aufweist, das für Gewebe nicht toxisch ist und Elektrizität isoliert,
- ein Anhaftungsteil (4),
- Elektronik zur Verarbeitung der Signale,
**dadurch gekennzeichnet, dass**
- das Kontaktelektrodenteil (1) kugelförmig ist,
- das elektrisch leitende Teil des Kontaktelektrodenteils (1)
durch eine elektronische Schaltung in dem Kontaktelektrodenteil (1) an das elektrisch leitende Innenteil (2a) des flexiblen leitenden Drahtes (2) angeschlossen ist,
- das Anhaftungsteil (4) imstande ist, das Elektrodenteil an der Person oder dem Tier angehaftet zu halten, und
- das Anhaftungsteil (4) durch eine elektronische Schaltung an den leitenden Draht (2) angeschlossen ist.

2. Elektrodendetektor nach Anspruch 1, **dadurch gekennzeichnet, dass** das Innenteil (1a) des Kontaktelektrodenteils (1) mehr als ein separates Material umfasst.

3. Elektrodendetektor nach Anspruch 1, **dadurch gekennzeichnet, dass** in dem Anhaftungsteil (4) eine oder mehr elektrische Kopplung(en) vorhanden ist (sind).

4. Elektrodendetektor nach Anspruch 1, **dadurch gekennzeichnet, dass** in dem Anhaftungsteil (4) eine oder mehr elektrische Kopplung(en) vorhanden ist (sind), die das Abdeckteil (2b) des leitenden Drahtes (2) erfassen.

5. Elektrodendetektor nach Anspruch 1, **dadurch gekennzeichnet, dass** in dem Abdeckteil (2b) des leitenden Drahtes (2), das an dem Kontaktelektrodenteil (1) befestigt ist, ein oder mehr Befestigungselement(e) vorhanden ist (sind).

6. Elektrodendetektor nach Anspruch 1, **dadurch gekennzeichnet, dass** in dem Anhaftungsteil (4) ein oder mehr Anhaftungselement(e) vorhanden ist (sind).

7. Elektrodendetektor nach Anspruch 1 und 6, **dadurch gekennzeichnet, dass** ein oder mehr Anhaftungselement(e) des Anhaftungsteils (4) aus flexiblem, gelatineartigen Material, wie Gummi, sind.

8. Elektrodendetektor nach Anspruch 6 und 7, **dadurch gekennzeichnet, dass** das eine oder die mehreren Anhaftungselement(e) des Anhaftungsteils (4) aus flexiblem, gelatineartigen Material sind, das den gesamten Elektrodendetektor oder einen Teil davon bedeckt.

9. Elektrodendetektor nach Anspruch 1, **dadurch gekennzeichnet, dass** die elektronische Schaltung im Inneren des Anhaftungsteils (4) ein Differenzialverstärker ist.

10. Elektrodendetektor nach Anspruch 1, **dadurch gekennzeichnet, dass** die elektronische Schaltung im Inneren des Kontaktelektrodenteils (1) ein Differenzialverstärker ist.

11. Elektrodendetektor nach Anspruch 1, **dadurch gekennzeichnet, dass** das Anhaftungsteil (4) des Elektrodendetektors ein Elektrizität isolierendes, nicht toxisches Material ist.

12. Elektrodendetektor nach Anspruch 1, **dadurch gekennzeichnet, dass** dieser zusätzliche Kontaktelektrodenteile (1), leitende Drähte (2) und Anhaftungsteile (4) umfasst.

## Revendications

1. Détecteur d'électrode, qui comprend :
- une partie d'électrode de contact (1) pour surveiller des bio-potentiels sur un oeil d'une personne ou sur un oeil d'un animal, la partie d'électrode de contact (1) comprenant une partie de recouvrement (1b) qui est d'une matière non toxique pour les tissus et une partie électro-conductrice,
- un fil conducteur flexible (2) attaché à la partie d'électrode de contact (1), le fil conducteur flexible (2) ayant une partie intérieure électro-conductrice (2a) et une partie de recouvrement (2b) qui n'est pas toxique pour les tissus et isole de l'électricité,
- une partie d'attachement (4),
- des composants électroniques pour traiter les signaux,
**caractérisé en ce que** :
- la partie d'électrode de contact (1) est en forme de bille,
- la partie électro-conductrice de la partie d'électrode de contact (1) est, de manière à travers un circuit électronique dans la partie d'électrode de contact (1), connectée à la partie intérieure électro-conductrice (2a) du fil conducteur flexible (2),
- la partie d'attachement (4) est capable de maintenir la partie d'électrode attachée à la personne ou à l'animal, et
- la partie d'attachement (4) est connectée, de manière à travers un circuit électronique au fil conducteur (2).

2. Détecteur d'électrode selon la revendication 1, **caractérisé en ce que** la partie intérieure (1a) de la partie d'électrode de contact (1) comprend plus d'une matière distincte.

3. Détecteur d'électrode selon la revendication 1, **caractérisé en ce que** la partie d'attachement (4) contient un ou plusieurs accouplements électriques.

4. Détecteur d'électrode selon la revendication 1, **caractérisé en ce que** la partie d'attachement (4) contient un ou plusieurs accouplements s'accrochant à la partie de recouvrement (2b) du fil conducteur (2).

5. Détecteur d'électrode selon la revendication 1, **caractérisé en ce que** la partie de recouvrement (2b) du fil conducteur (2) attachée à la partie d'électrode de contact (1) contient un ou plusieurs éléments d'attachement.

6. Détecteur d'électrode selon la revendication 1, **caractérisé en ce que** la partie d'attachement (4) contient un ou plusieurs éléments d'attachement.

7. Détecteur d'électrode selon les revendications 1 et 6, **caractérisé en ce qu'**un ou plusieurs éléments d'attachement de la partie d'attachement (4) sont d'une matière gélatineuse flexible, comme du caoutchouc.

8. Détecteur d'électrode selon les revendications 6 et 7, **caractérisé en ce que** lesdits un ou plusieurs éléments d'attachement de la partie d'attachement (4) sont d'une matière gélatineuse flexible qui recouvre l'intégralité ou une partie du détecteur d'électrode.

9. Détecteur d'électrode selon la revendication 1, **caractérisé en ce que** le circuit électronique à l'intérieur de la partie d'attachement (4) est un amplificateur différentiel.

10. Détecteur d'électrode selon la revendication 1, **caractérisé en ce que** le circuit électronique à l'intérieur de la partie d'électrode de contact (1) est un amplificateur différentiel.

11. Détecteur d'électrode selon la revendication 1, **caractérisé en ce que** la partie d'attachement (4) du détecteur d'électrode est une matière non toxique d'isolation électrique.

12. Détecteur d'électrode selon la revendication 1, **caractérisé en ce qu'**il comprend des parties d'électrode de contact (1), des fils conducteurs (2) et des parties d'attachement (4) supplémentaires.
